# EUROPEAN PATENT SPECIFICATION

(11) **EP 1 722 624 B1**
(45) Date of publication and mention of the grant of the patent: **15.10.2014**
(21) Application number: 04748401.9
(22) Date of filing: 09.07.2004
(51) Int. Cl.: A01H 4/00, C12N 15/82

(54) **METHOD FOR PREPARING TRANSGENIC PEPPER USING CALLUS INDUCTION**
VERFAHREN ZUR HERSTELLUNG VON TRANSGENEM CAPSICUM MITTELS KALLUSINDUKTION
PROCEDE POUR PRODUIRE DES PIMENTS TRANSGENIQUES PAR CALLOGENESE

(30) Priority: 12.03.2004 KR 2004016722
(43) Date of publication of application: 22.11.2006
(73) Proprietor: NONGWOOBIO, Suwon-si Gyeonggi-do 443-372 (KR)
(72) Inventor: HARN, Chee Hark, Incheon-si, Gyeonggi-do 467-854 (KR); LEE, Yun Hee, Gyeonggi-do 469-885 (KR); KIM, Ju Yeon, Gyeonggi-do 469-885 (KR); KIM, Hyo Soon, Gyeonggi-do 469-885 (KR); JUNG, Min, Gyeonggi-do 469-885 (KR); CHOI, Soon Ho, Gyeonggi-do 469-885 (KR); YANG, Seung Gyun, Gyeonggi-do 469-885 (KR)
(74) Representative: Hiebl, Inge Elisabeth
(86) International application number: PCT/KR2004/001686
(87) International publication number: WO 2005/086993

(56) References cited:
- US-A- 5 262 316
- LEE Y H ET AL: "A new selection method for pepper transformation: callus-mediated shoot formation" PLANT CELL REPORTS, vol. 23, no. 1-2, August 2004 (2004-08), pages 50-58, XP002447658 ISSN: 0721-7714
- LI D ET AL: "Establishment of a highly efficient transformation system for pepper (Capsicum annuum L.)." PLANT CELL REPORTS, vol. 21, no. 8, April 2003 (2003-04), pages 785-788, XP002447659 ISSN: 0721-7714
- CAI, W.Q. & AL: ""Virus-resistant chili pepper produced by agrobacterium species-mediated transformation " in "Transgenic plants and crops ": (pages 563-578)" 2002, KHACHATOURIANS, G.G. MCHUGHEN, A. SCORZA, R. NIP, W.K. HUI, Y.H. , NEW YORK , XP009088515 Materials and Methods, section B
- LEE SANG JIK ET AL: "PPI1: A novel pathogen-induced basic region-leucine zipper (bZIP) transcription factor from pepper" MOLECULAR PLANT-MICROBE INTERACTIONS, vol. 15, no. 6, June 2002 (2002-06), pages 540-548, XP002447660 ISSN: 0894-0282
- MANOHARAN M. ET AL: 'Agrobacterium-mediated genetic transformation in hot chilli (Capsicum annuum L. var. Pusa jwala).' PLANT SCIENCE. vol. 131, 1998, pages 77 - 83, XP003012243
- LIU W. ET AL: 'Agrobacterium induced gall formation in bell pepper (capsicum annuum L.) and formation of shoot-like structure expressing induced genes.' PLANT CELL REPORTS. vol. 9, 1990, pages 360 - 364, XP008080938
- LEE S.J. ET AL: 'In vitro plant regeneration and Agrobacterium-ediated transformation from cotyledon explant of hot pepper (Capsicum annuum L. cv. Golden Tower).' KOREAN J PLANT TISSUE CULTURE. vol. 20, 1993, pages 289 - 294, XP008081061
- ZHU Y.X. ET AL: 'Transgenic sweed peppers plants from Agrobacterium-mediated transformation.' PLANT CELL REPORTS. vol. 16, 1996, pages 71 - 75, XP008080922

## Description

### TECHNICAL FIELD

The present invention relates to a method for producing transgenic pepper plants, and more particularly to a method for mass-producing transgenic pepper plants using callus induction, the method being characterized by the steps of: pre-culturing pepper explants; co-culturing the pre-cultured pepper explants with *Agrobacterium* into which a target gene have been introduced; selecting callus; and forming shoots.

### BACKGROUND ART

Pepper is one of the main crops in the world and widely used as food or seasonings. Also, pepper is used in industrial dyes, particularly it is used as a main raw material of cosmetic lipsticks. The total annual sales amount of pepper products in Korea is about two trillion won in Korean currency and forms the largest seed market in Korea. Pepper is the most suitable crop to be able to create high added value in relation to the use of biotechnology.

The first gateway to biotechnically use pepper is to perform transformation. However, pepper is known as a crop which is very difficult to be transformed due to the following two reasons. First, the success probability of regeneration into transgenic pepper plants definitely varies depending on seed lines (Christopher, T et al., Plant Cell Tiss. Org. Cult., 46:245, 1996; Kim, JY et al., J. Plant Biotechnol., 4:129, 2002). Since high transformation rate is obtained only in seed lines with high regeneration rate, it is very difficult for seed lines with low regeneration rate to be transformed. Second, the infection rate of *Agrobacterium* varies depending on crops. It is inferred that crops, which are difficult to be transformed, are the case where the pilus of *Agrobacterium* is impermeable to the tissue cell of explants or not sufficiently inoculated to the tissue cell.

The cases of success in the transformation of pepper are still rare throughout the world. A method for transforming pepper plants using *Agrobacterium* was first reported in USA (US Patent No. 5,262,316), and also studied by Sung-Han Son, et al. in Korea (Korean Patent Registration No. 344573). However, such methods have disadvantages in that they show a very low transformation rate, are not reproducible and make it difficult to mass-produce transgenic pepper plants.

### DISCLOSURE OF INVENTION

During our extensive studies to mass-produce transgenic pepper plants at high efficiency, the present inventors have found that, when explants of pepper are regenerated by pre-culture in callus induction medium followed by co-culture with *Agrobacterium,* transgenic pepper can be easily obtained, thereby completing the present invention.

Accordingly, a main object of the present invention is to provide a method for mass-producing transgenic pepper plants, the method being characterized by pre-culturing explants of pepper in callus induction medium, followed by co-culturing with *Agrobacterium.*

To achieve the above object, the present invention provides a method for mass-producing transgenic pepper plants, the method comprising the steps of: (a) pre-culturing explants of pepper in a callus induction medium; (b) co-culturing the pre-cultured explants with *Agrobacterium* into which a target gene have been introduced; (c) culturing the co-cultured explants in a selection medium so as to form callus and selecting the formed callus; and (d) cutting the callus and culturing the cut callus in a shoot induction medium so as to form shoots.

As used herein, the term "explants" refers to tissue segments excised from plants, which includes cotyledons or hypocotyls.

Hereinafter, the present invention will be described in detail.

The method of the present invention comprises the following steps: (a) the pre-culture of pepper explants; (b) transformation (co-culture with *Agrobacterium* into which a target gene have been introduced), (c) callus selection; and (d) shoot formation. Also, the inventive method may additionally comprise the steps of root formation and soil adaptation. Particularly, the inventive method is characterized by that pepper explants are pre-cultured in callus induction medium and transformed so as to artificially form callus, and the regeneration of plants is induced from the formed callus. Each step of the inventive method will now be described in detail.

### (a) Pre-culture of explants for callus induction

In the inventive method, explants of pepper are first pre-cultured in callus induction medium. At this time, the explants are preferably wounded in order to promote callus induction. Furthermore, as the pepper explants, cotyledons or hypocotyls can be used in the present invention. Cotyledons obtained by sterilizing and washing pepper seeds and germinating the washed seeds in MS medium are more preferably used.

The callus induction medium which is used in the pre-culture step of the inventive method refers to a plant culture medium (e.g., MS medium and 1/2 MS medium) containing a plant hormone that induces callus. The plant hormone which can be used for callus induction is one selected from the group consisting of zeatin, 2,4-dichlorophenoxy acetic acid (2,4-D), indole-3-acetic acid (IAA), naphthalene acetic acid (NAA) and benzylaminopurine (BA).

Each of the plant hormones is added at the following concentration: 0.01-5 mg/l of 2,4-D, 0.01-5 mg/l of IAA, 0.01-5 mg/l of zeatin, 0.01-5 mg/l of NAA, and 0.01-5 mg/l of BA. Most preferably, 1 mg/l of 2,4-D or 1 mg/l of IAA may be used. A mixture of the above-mentioned hormones may also be used. Preferably, a mixture of 0.01-2 mg/l of zeatin with one selected from the group consisting of 0.1-5 mg/l of 2,4-D, 0.1-5 mg/l of IAA and 0.01-2 mg/l of NAA may be used. The pre-culture step is preferably performed at 22-28°C for 20-60 hours. During the pre-culture step, callus is induced at the wounded parts of the explants by the influence of the hormone.

### (b) Transformation (co-culture with Agrobacterium into which a target gene have been introduced)

In order to transform the explants which have been pre-cultured in the callus induction medium, the pre-cultured explants are co-cultured with *Agrobacterium.* Before the co-culture, a target gene to be introduced into pepper plants needs to be introduced into *Agrobacterium.* The transformation of *Agrobacterium* may be performed by any method known in the art. For example, the freeze-thaw method (Glevin et al., Plant molecular biology. Kluwer Academic Publishers A3/7, 1988) may be used. Thereafter, *Agrobacterium* into which the target gene have been introduced is inoculated to the pre-cultured pepper explants for 10-20 minutes and then co-cultured. At this time, the same medium as the callus induction medium used in the step (a) is preferably used as co-culture medium. Moreover, the co-culture step is preferably performed at 22-28°C for 40-80 hours.

### (c) Callus selection

The pepper explants which have been co-cultured with *Agrobacterium* are cultured in selection medium to select transgenic callus. The selection medium preferably contains not only an antibiotic for the selection of transgenic callus but also zeatin and IAA for the induction of callus development. As a substitute for IAA, NAA may also be used. Furthermore, the antibiotic used in the callus selection is preferably an antibiotic corresponding to an antibiotic-resistant gene present in a recombinant vector introduced into *Agrobacterium.* Also, zeatin is preferably used at a concentration of 0.1-5 mg/l, each of IAA and NAA is preferably used at a concentration of 0.01-1 mg/l. More preferably, a mixture of 2 mg/l of zeatin and 0.3 mg/l of IAA may be used. 4-5 weeks after the explants co-cultured with Agrobacterium are transferred to the selection medium, callus begins to grow. Then, the callus is preferably cultured for additional 5-8 weeks.

### (d) Shoot formation

The transformed callus obtained in the step (c) is cut into small pieces. The cut callus pieces are transferred to shoot induction medium to induce shoot formation. The shoot induction medium preferably contains zeatin alone or a mixture of zeatin and one of IAA and NAA. Zeatin is preferably used at a concentration of 0.5-10 mg/l, and each of IAA and NAA is preferably used at a concentration of 0.01-0.2 mg/l. More preferably, a mixture of 2 mg/l of zeatin and 0.01 mg/l of IAA may be used. One month after the callus is transferred to the shoot induction medium, shoots are formed. Then, the shoots are preferably cultured in the same medium for an additional period of about two months, so as to elongate the shoots.

### (e) Root formation and soil adaptation

The elongated shoots are transferred and cultured in root induction medium. The root induction medium preferably contains no plant hormone. Preferably, a MS basal medium containing a small amount of antibiotics may be used as the root induction medium. About 4-5 weeks after transfer to the root induction medium, roots are formed. When the roots grow about 4-10 cm, the medium is clearly removed and the roots are planted in pots.

The inventive method for transforming pepper plants has the following advantages.

First, direct shoot induction from explants of pepper according to the prior *Agrobacterium-*mediated transformation method provides little or no transformation, whereas the inventive method shows remarkably high transformation rate.

Second, the inventive method is not limited to certain lines of pepper plants (line non-specific). Thus, the inventive method can stably transform various lines of pepper plants.

Third, the inventive method can mass-produce transgenic plants within a shorter time period than that of the prior method, since the callus induction hormone is added in the step of pre-culturing the explants so as to shorten the time for callus induction.

Accordingly, various useful genes, such as genes involved in plant defense mechanisms or genes involved in the biosynthesis of useful metabolites, can be introduced into pepper plants by the inventive transformation method, so that a variety of functional transgenic pepper plants can be mass-produced at high efficiency.

Example of the genes involved in plant defense mechanisms, which can be used in the present invention, are preferably genes encoding a protein selected from the group consisting of ribosome inactivating protein (RIP), jasmonic acid carboxyl methyltransferase, trehalose synthase, plant defensin 1.2, thionine synthase, glucanase, chitinase, phenylalanine ammonia lyase, chalcone synthase, glutathione-*S*-transferase, anthranilate synthase, storage protein, calmodulin, tryptophan synthase, proteinase inhibitor II, nitric oxide synthase, cystemine, fatty acid peroxidase, allene oxide synthase, pepper-PMMV interaction 1 transcription factor (PPI1), WRKY domain transcription factor, pathogen-responsive protein, and virus coat protein, but are not limited thereto. Examples of the genes encoding the virus coat proteins include *TMV-CP, CMV-CP* and *PepMoV-CP* genes.

Furthermore, the genes involved in the biosynthesis of useful metabolites are preferably selected from the group consisting of genes involved in the biosynthesis of tannin, sinapine, saponin, allicin, spinosin, cinnamic acid, flavonoid, terpenoid, catechin, vitamin, penicilline, indole, insulin, prostaglandin, taxol, alisol, ricin, cartenoid and capsisin, but are not limited thereto.

The inventive transformation method can be applied to all *Capsicum* genus peppers, and preferably *Capsicum annuum* L. Examples of *Capsicum annuum* L. include chilli pepper *(Capsicum annuum* L. var. acuminatum), sweet or bell pepper *(Capsicum annuum* L. var. grossum), cone pepper *(Capsicum annuum* L. var. conoides), cherry pepper *(Capsicum annuum* L. var. cerasiforme), red cluster pepper (*Capsicum annuum* L. var. fasciculatum), long pepper *(Capsicum annuum* L. var. longum), and so on.

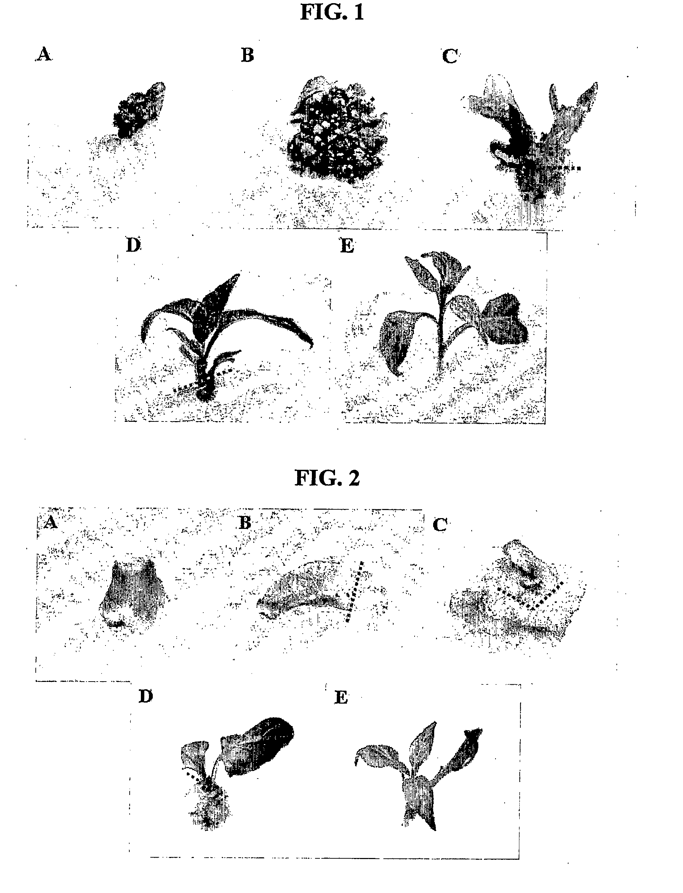

In one embodiment of the present invention, explants of pepper were pre-cultured in a medium containing either a mixture of zeatin and NAA or a mixture of zeatin and IAA, and then transformed with *Agrobacterium* into which a *TMV-CP* gene or a *PPI1* gene have been introduced. Thereafter, unlike a general case where shoots are formed directly from wounded parts of transformed explants during a regeneration process of pepper (direct shoot formation), it was observed that there was a case where callus had been induced in wounded parts, from which shoots have then been formed (indirect shoot formation; callus-mediated shoot formation) (see **FIG. 1** and **FIG. 2****).**

The transformation rates in the above two cases were examined and the results showed that, in the case of direct shoot formation, the frequency of direct shoot formation was high, but the frequency of root formation from shoots was low and none of the individual shoots were transformed (see **Table 1** and **Table 3).** On the other hand, in the case of indirect shoot formation mediated by callus, although the frequency of callus induction from the wounded parts of explants was very low, the frequency of shoot formation from the induced callus and root formation from the shoots were high (see **Table 2).** Furthermore, the transformation rates of the shoots formed from the callus were examined and the results showed that the transformation rates were 0.19% for P915 line pepper and 0.03% (3/37,500) for P409 line pepper (see **Table 4**)**.** From such results, the present inventors could find that a high transformation efficiency of pepper is achieved in the case where shoot formation is induced from transformed callus. This was first found in the present invention.

Therefore, the present invention provides transgenic pepper plants transformed with a gene encoding a PPI1 protein, as well as TMV-resistant pepper plants transformed with a gene encoding TMV-CP.

In another embodiment of the present invention, in order to more efficiently induce callus from explants of pepper, the pepper explants were pre-cultured in a medium containing one or more of various plant hormones and then co-cultured with *Agrobacterium* into which a *GFP* gene have been introduced. At this time, 2,4-D, IAA or a mixture of zeatin and 2,4-D were used as the plant hormone, respectively. Thereafter, the transformed explants were cultured in selection medium to select transgenic callus (see **FIG. 3** and **FIG. 4**). The callus was cut into small pieces and then transferred to shoot induction medium to form shoots (see **FIG. 5**). Following this, roots were induced from the shoots and then regenerated into complete plants via an adaptation step (see **FIG. 6**).

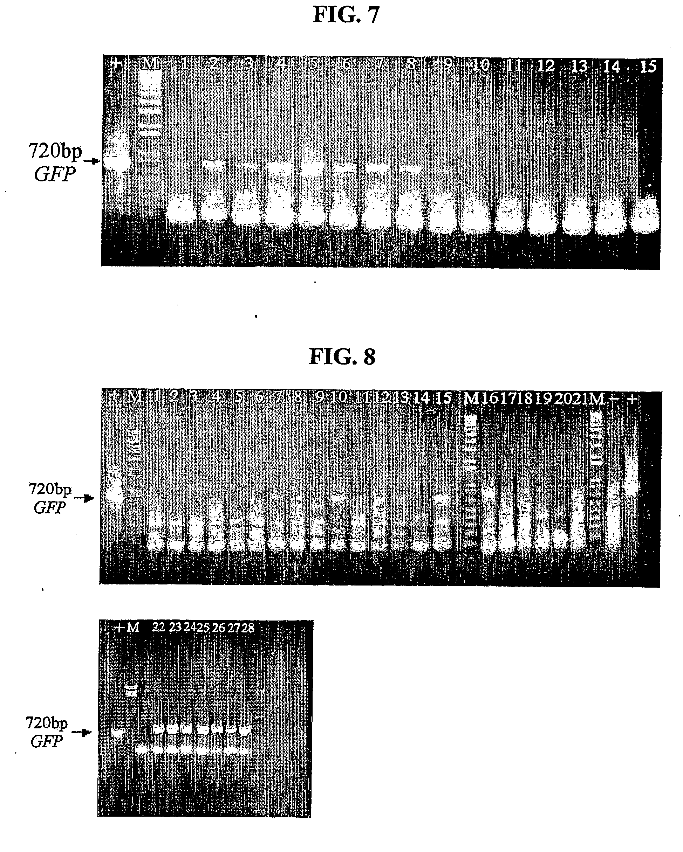

The callus induction rates in the case where the pepper explants have been pre-cultured in the medium containing a mixture of 2,4-D and zeatin, 2,4-D or IAA was examined. The results showed that the callus was generally induced at a rate of about 13%, although there is a slight difference in callus induction rate between pepper lines (see **Table 6**). This callus induction rate is about 10 times higher than that of the case where explants were pre-cultured in a medium containing zeatin and NAA (or IAA). Furthermore, the transformation rate of shoots formed from callus was about 1%, based on the total number of explants used in transformation (see **FIG 7**). This value is about 5 times higher than the callus-mediated shoot transformation rate (0.19%) of the case where explants were pre-cultured in a medium containing zeatin and NAA (or IAA). Moreover, in the case where the explants were pre-cultured in the medium containing the mixture of 2,4-D and zeatin, 2,4-D or IAA, it was found that all of 8 line peppers used in a test were stably transformed (see **Table 6** and **FIG. 7** to **FIG. 9**). The inventive method as described above allowed the transformation time to be shortened as compared to the prior methods by pre-culturing the pepper explants in the callus induction medium.

### BBIEF DESCRIPTION OF DRAWINGS

**FIG. 1** is a photograph showing each step of a regeneration process by direct shoot formation. The dotted lines represent parts cut for culture in a subsequent step (A: direct shoot formation; B: multi-shoot formation; C: multi-shoot elongation; D: single-shoot elongation from a multi-shoot; and E: root formation).
**FIG.** 2 is a photograph showing each step of a regeneration process by indirect shoot formation mediated by callus. The dotted lines represent parts cut for culture in a subsequent step (A: callus formation; B: callus development; C: shoot formation; D: single-shoot elongation; and E: root formation).

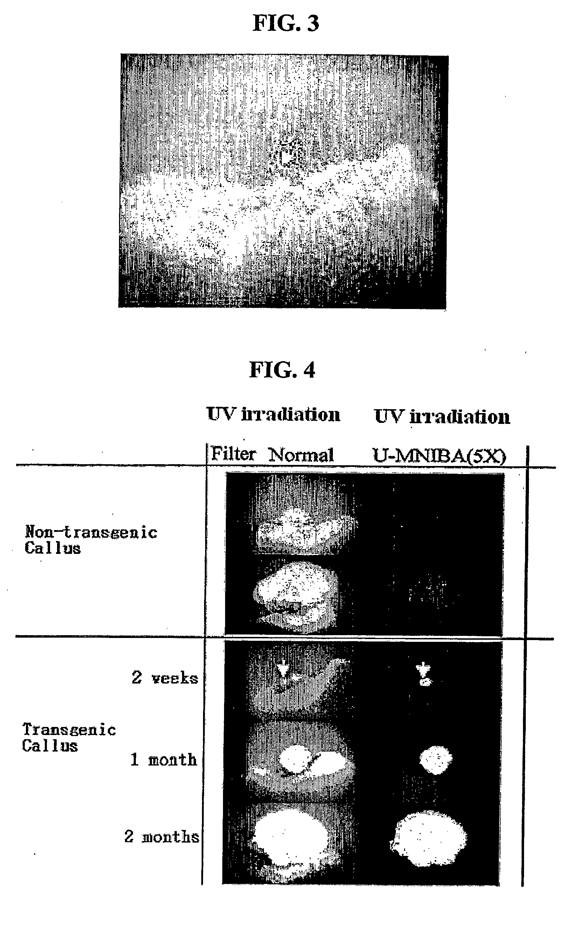

**FIG. 3** is a photograph showing callus (represented by the arrow) grown in selection medium for two weeks according to the present invention.
**FIG. 4** shows a state where callus transformed by the inventive method continuously expresses GFP during its growth.

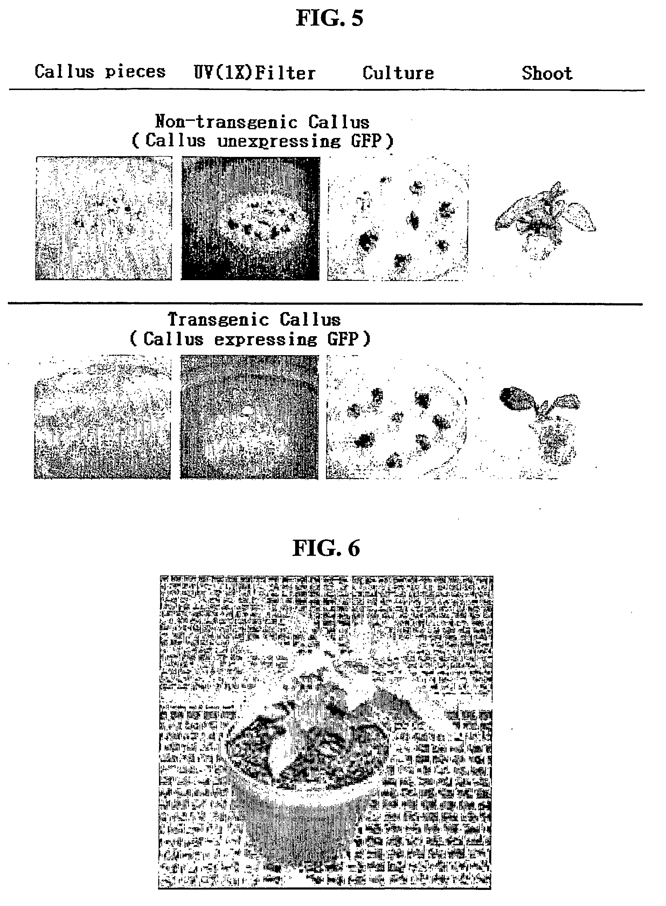

**FIG. 5** shows a process of shoot formation from callus transformed by the inventive method.
**FIG. 6** shows the appearance of a transgenic pepper plant according to the present invention, one month after it was planted in a pot after root formation.
**FIG. 7** shows the results of PCR amplification of genomic DNAs extracted from callus expressing GFP (lanes 1-9) and from callus expressing no GFP (lanes 10-15) (+: GFP clone; and M: molecular weight marker).
**FIG. 8** shows the results of PCR amplification of genomic DNAs extracted from the leaves of seedling plants regenerated from callus expressing GFP (+: GFP clone; M: molecular weight marker; lanes 1-9: P410 line; lanes 10-15: P915 line; lanes 16-21: P318 line; lanes 22-28: P319 line; and -: non-transgenic plant).

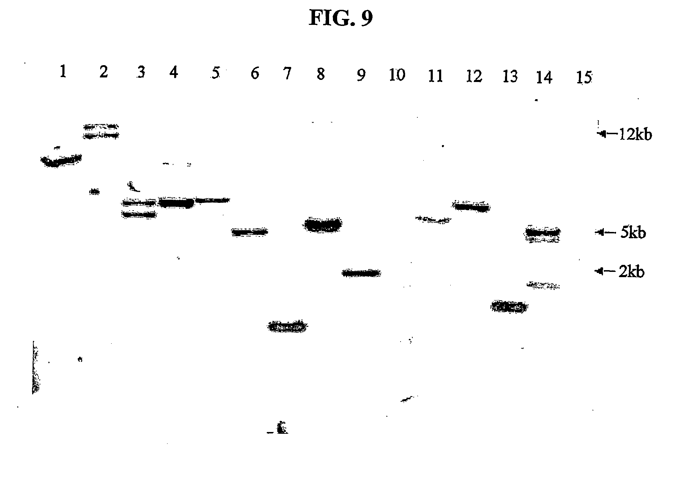

**FIG 9** shows the results of Southern blot analysis on genomic DNAs obtained from transgenic pepper plants produced by the inventive method (lanes 1-2: P915 line; lanes 3-6: P318 line; lanes 7-14: P319 line; and lane 15: non-transgenic plant).

### DETAILED DESCRIPTION OF THE INVENTION

The present invention will hereinafter be described in further detail by examples. It will however be obvious to a person skilled in the art that the present invention is not limited to or by the examples.

### Example 1: Production of pepper plants transformed with TMV-CP gene or PPI1 gene

### 1-1: Preparation and pre-culture of explants

Four inbred lines (P915, P409, P410 and P101) of chilli pepper seeds (Nongwoo Bio Co., Korea) were used in a test. The surface of the seeds was sterilized with 95% ethanol for 30 seconds and then with 50% bleach (Yuhanrocks, Yuhan Corp., Korea) for 10 minutes. Then, the seeds were washed three times with sterilized water. The sterilized seeds were transferred to 1/2 MS medium (1/2 MS, 1.5% sucrose, 0.8% agar, pH 5.8) and germinated under light conditions at 25°C. Cotyledons and hypocotyls were excised from the 8-10 day-old plants and used as explants. About 190,000 explants were wounded with a knife, after which they were transferred to pre-culture medium (MS basal medium containing 2 mg/l zeatin and 0.05 mg/l NAA or 0.1 mg/l IAA) and cultured under light conditions at 22-28°C for 2-36 hours.

### 1-2: Co-culture with Agrobacterium

A DNA fragment encoding a *TMV-CP* gene (GenBank accession No: L35074; *Park et al.,* 1997) or a *PPI1* gene (GenBank accession No: AF430372; Lee *et al.,* 2002) was inserted into a pCAMBIA 2300 vector (CAMBIA, Australia). The resulting recombinant vector was introduced into an *Agrobacterium* LBA4404 strain or EHA105 strain. The transformed *Agrobacterium* strain was cultured in a YEP medium containing 50mg/l kanamycin, 50mg/l rifampicin and 100 µM acetosyringone. The culture broth was centrifuged and then diluted in MS basal medium to an OD₆₀₀ of 0.3-0.5. The culture suspension was mixed with a MS basal medium containing 100 µM acetosyringone, and the mixture was inoculated to the explants pre-cultured in Example 1-1 above for 10-20 minutes. Thereafter, the explants were co-cultured with the *Agrobacterium* strain in a medium having the same composition as that of the pre-culture medium used in Example 1-1 above, under dark conditions for 38-96 hours. The explants co-cultured with the *Agrobacterium* strain were washed three times with a 1/2 MS liquid medium containing 500∼800 mg/l cefotaxime or lilacilline.

### 1-3: Shoot formation

In order to select transgenic plants, the explants co-cultured with the *Agrobacterium* strain in Example 1-2 above were cultured in a MS basal medium (selection medium) containing 2 mg/l zeatin, 0.05 mg/l NAA (or 0.1 mg/l IAA), 80-100 mg/l kanamycin and 300 mg/l cefotaxime (or lilacilline) for 6-8 weeks. 4-5 weeks after the culture, it could be observed that tissue similar to callus was formed around the wounded parts of several explants. Thereafter, in order to induce shoots, the explants were transferred to a MS basal medium (elongation medium) containing 2 mg/l zeatin, 0.01 mg/l NAA (or 0.01 mg/l IAA), 60-100 mg/l kanamycin and 300mg/l cefotaxime and cultured for 7-10 weeks.

### 1-4: Root formation and soil adaptation

The elongated shoots were transferred to a MS basal medium (root induction medium) containing 20-30 mg/l kanamycin and 200 mg/l cefotaxime and cultured for 6-8 weeks. The regenerated plants were transferred into a zippy pot and cultured under a 16 hr light condition at 25°C for 2 weeks.

In the above test, it could be confirmed that two patterns of shoot formation from the explants appeared. One pattern is a general pattern where shoots (or multiple shoots) are formed directly from the wounded parts of the explants (direct shoot formation), and the other pattern is one where callus tissue is formed around the wounded parts of the explants, and then, shoots are formed from the callus tissue (indirect shoot formation; callus-mediated shoot formation).

The regeneration process by the direct shoot formation was observed in the most cases. As shown in **FIG. 1****,** the regeneration process consisted of 5 steps including shoot formation, multi-shoot formation, multi-shoot elongation, single-shoot elongation, and root formation. On the other hand, the regeneration process by the indirect shoot formation mediated by callus was very rarely observed, and this is believed that it is because of callus not being easy to be naturally induced from the wounded parts of cotyledons. As shown in **FIG. 2****,** the regeneration process by the indirect shoot formation consisted of five steps including callus formation, callus development, shoot formation, single-shoot elongation and root formation.

### Example 2: Examination of shoot formation frequency

### 2-1: Frequency of direct shoot formation

A total of 151,700 explants obtained from four lines of pepper plants were transformed in the same manner as in Example 1, after which the frequency of direct shoot formation from the wounded parts of the explants was examined. The frequency of direct shoot formation on the shoot induction medium was 5.3% (8,089/151,700) (see **Table 1**). However, the rate of survived shoots on the root induction medium was 17.4% (1,407/8,089). The numerical values for each plant line set forth in **Tables 1 and 2** represent the total of results for each of hormones used.

**Table 1: Frequency of direct shoot formation**

| Genes | Number of explants | | | | Number of shoots | | | | Number of shoots with root | | | |
|---|---|---|---|---|---|---|---|---|---|---|---|---|
| | P915 | P409 | P410 | P101 | P915 | P409 | P410 | P101 | P915 | P409 | P410 | P101 |
| *TMV-CP* | 30,512 | 26,039 | 24,107 | 21,983 | 2,106 | 1,017 | 1,697 | 628 | 392 | 156 | 311 | 49 |
| *PPI1* | 14,413 | 14,080 | 7,488 | 13,078 | 1,024 | 587 | 720 | 310 | 186 | 103 | 176 | 34 |
| Subtotal | 44,925 | 40,119 | 31,595 | 35,061 | 3,130 | 1,604 | 2,417 | 938 | 578 | 259 | 487 | 83 |
| Total | 151,700 | | | | 8,089 | | | | 1,407 | | | |

### 2-2: Frequency of indirect shoot formation mediated by callus

A total of 37,500 explants obtained from four lines of pepper plants were transformed in the same manner as in Example 1 above, after which the frequency of callus-mediated shoot formation was examined. The frequency of callus formation from the explants was a very low level of 1.2% (459/37,500) (see **Table 2**). However, the frequency of shoot development from the callus was 11.6% (53/459), and the frequency of root formation from the shoots was 52.8% (28/53). These are values that are much higher than the rates of shoot and root formation in the regeneration process by the direct shoot formation.

**Table 2: Frequencies of callus development and shoot formation from callus**

| Genes | Number of explants | | | | Number of callus formed | | | | Number of shoots formed from the callus | | | | Number of shoots with roots | | | |
|---|---|---|---|---|---|---|---|---|---|---|---|---|---|---|---|---|
| | P915 | P409 | P410 | P101 | P915 | P409 | P410 | P101 | P915 | P409 | P410 | P101 | P915 | P409 | P410 | P101 |
| TMV-CP | 5,188 | 6,917 | 5,491 | 7,210 | 81 | 30 | 25 | 94 | 14 | 5 | 1 | 7 | 12 | 3 | 0 | 2 |
| PPI1 | 2,903 | 3,056 | 3,798 | 2,937 | 107 | 46 | 17 | 59 | 15 | 4 | 2 | 5 | 7 | 2 | 1 | 1 |
| Sub-total | 8,091 | 9,973 | 9,289 | 10,147 | 188 | 76 | 42 | 153 | 29 | 9 | 3 | 12 | 19 | 5 | 1 | 3 |
| Total | 37,500 | | | | 459 | | | | 53 | | | | 28 | | | |

The above results suggest that, once callus is formed in the regeneration of pepper explants, shoots and roots can be formed from the callus at high efficiency.

### Example 3: Examination of transformation efficiency

A total of 1,407 shoots formed directly from the wounded parts of explants in Example 1, and a total of 28 shoots formed indirectly from callus, were examined for their transformation rates by PCR assay.

First, the genomic DNA of pepper plants was isolated according to the method described in Lee et al., Plant Mol. Biol., 46:661, 2001. To detect the insertion of a *TMV-CP* gene, PCR was performed using primers represented by SEQ ID NO: 1 and SEQ ID NO: 2. Furthermore, to detect the insertion of a *PPI1* gene, PCR was performed using primers represented by SEQ ID NO: 3 and SEQ ID NO: 4. PCR was performed by repeating 35 cycles, each consisting of 1 minute at 94°C, 1 minute at 55°C and 1 minute at 72°C.

The PCR assay results showed that none of the 1,407 shoots formed directly from the wounded parts of explants were inserted with the *TMV-CP* gene or *PPI1* gene (see **Table 3**). Transformation efficiencies set forth in **Tables 3 and 4** below are values obtained by dividing the number of shoots which are positive in the PCR assay by the number of explants used.

**Table 3: Transformation rates of shoots formed directly from wounded parts of explants**

| Genes | Number of shoots positive in the PCR assay | | | |
|---|---|---|---|---|
| | P915 | P409 | P410 | P101 |
| *TMV-CP* | 0 | 0 | 0 | 0 |
| *PPI1* | 0 | 0 | 0 | 0 |
| Total | 0 | 0 | 0 | 0 |
| Transformation efficiency | 0% | 0% | 0% | 0% |

On the other hand, examination results for the transformation rates of shoots formed indirectly from callus showed that the transformation rates were 0.19% (15/37,500) for P915 line plants and 0.03% (3/37,500) for P409 line plants (see **Table 4**). The ratio of the number of shoots which are positive in the PCR assay to the number of shoots formed from callus was 34% (18/53).

**Table 4: Transformation rates of shoots formed indirectly from callus**

| Genes | Number of shoots positive in the PCR assay | | | |
|---|---|---|---|---|
| | P915 | P409 | P410 | P101 |
| *TMV-CP* | 10 | 2 | 0 | 0 |
| *PPI1* | 5 | 1 | 0 | 0 |
| Total | 15 | 3 | 0 | 0 |
| Transformation efficiency | 0.19% | 0.03% | 0% | 0% |

The above results suggest that, if shoots are formed from callus after transformation, the possibility of transformation of pepper plants is greatly increased.

### Example 4: Examination of TMV resistance of transgenic pepper plants

In order to examine if the transgenic pepper plants into which the *TMV-CP* gene have been introduced by the indirect shoot formation mediated by callus in Example 1 above shows resistance to TMV, the following test was carried out. T0 plants were self-crossed to obtain T1 plants. The leaves of 408 individuals of the T1 plants were inoculated with TMV twice at intervals of two-weeks. Two weeks after the second inoculation, ELISA was performed using a TMV-CP antibody (Shin, R. et al., Mol. Planet Microbe. Interact., 15:983, 2002). The results showed that 28 individuals of 408 individuals of the T1 plants were resistant to TMV infection (see **Table 5**). Mosaic spots were observed in the leaves of susceptible plants, but not observed in the resistant plants.

Furthermore, a genomic DNA template isolated from the leaves of plants was subjected to PCR in the same manner as in Example 3. The PCR assay results showed that the *TMV-CP* gene was inserted into all of 28 individual resistant plants (data not shown).

**Table 5: Examination of TMV resistance**

| | Number of plants tested | Number of susceptible plants | Number of resistant plants |
|---|---|---|---|
| T1 | 408 | 380 | 28 |
| Non-transformant | 66 | 66 | 0 |

### Example 5: Production of pepper plants transformed with GFP gene

### 5-1: Preparation ofpre-culture of explants

In order to determine the optimal conditions to induce callus from explants of pepper at a higher level, the explants were pre-cultured in a medium containing one or more of various plant hormones.

First, eight inbred lines (P915, P318, P319, P409, P410, P784, P2377 and PMAL) of chilli pepper seeds (Nongwoo Bio Co., Korea) were used in a test. The surface of the seeds was sterilized with 95% ethanol for 30 seconds and then with 50% bleach (Yuhanrocks, Yuhan Corp., Korea) for 10 minutes. Then, the seeds were washed three times with sterilized water. The sterilized seeds were transferred to a 1/2 MS medium (1/2 MS + 1.5% sucrose + 0.8% agar, pH 5.8), and then germinated under light conditions at 25°C. Following this, cotyledons were excised from the 8-10 day-old plants. The cotyledons were wounded with a knife, after which they were transferred to a callus induction medium containing one or more of various plant hormones (MS basal medium containing 1 mg/l 2,4-D, 1 mg/l IAA or a mixture of 1 mg/12,4-D and 0.2 mg/l zeatin) and pre-cultured. The pre-culture was performed in a temperature-controlled incubator (22-28°C) under light conditions for 20-60 hours.

### 5-2: Co-culture with Agrobacterium

*A GFP* gene (GenBank accession No: AY508125; Haseloff J, et al., PNAS, 94:2122, 1997) was introduced into a pCAMBIA 2300 vector. The resulting recombinant vector was introduced into an *Agrobacterium* LBA4404 strain. The transformed *Agnobacterium* strain was cultured in a YEP medium containing 50mg/l kanamycin, 50mg/l rifampicin and 100 µM acetosyringone. The culture broth was centrifuged, and then diluted in MS basal medium to an OD₆₀₀ of 0.3-0.6. The culture suspension was mixed with a MS liquid medium containing 100 µM acetosyringone, and the mixture was inoculated to the cotyledons pre-cultured in Example 5-1 above for 20 minutes. Thereafter, the cotyledons were co-cultured with the *Agrobacterium* strain in a medium having the same composition as that of the pre-culture medium used in Example 5-1, under dark conditions for 48-70 hours. The cotyledons co-cultured with the *Agrobacterium* strain were washed three times with a 1/2 MS liquid medium containing 500-800 mg/l cefotaxime.

### 5-3: Callus selection

In order to select transgenic callus, the cotyledons co-cultured with the *Agrobacterium* strain in Example 5-2 above were transferred to a MS basal medium (selection medium) containing 2 mg/l zeatin, 0.3 mg/l IAA, 80 mg/l kanamycin, 100 mg/l cefotaxime and 300 mg/l lilacilline, and cultured in an incubator under a 16-hr light/8-hr dark cycle for 4-5 weeks. During this culture process, callus began to grow. **FIG. 3** is a photograph showing the callus grown for two weeks after transfer to the selection medium. Then, the callus was additionally cultured for 5-8 weeks. The growth pattern of the transgenic callus according to the culture time was observed under UV microscope and the results are shown in **FIG. 4****.** As shown in **FIG. 4****,** it could be confirmed that the transgenic callus expressing GFP shows continuous expression of GFP during its growth, thereby indicating that it is genetically stable.

### 5-4: Shoot formation

The grown callus was cut into small pieces, and then transferred to a MS basal medium (shoot induction medium) containing 2 mg/l zeatin, 0.01 mg/l IAA, 30-60 mg/l kanamycin and 300 mg/l cefotaxime and cultured for about one month. Thereafter, the formed shoots were additionally cultured for about two months so as to elongate the formed shoots. The process of shoot formation from the transformed callus is shown in **FIG. 5****.**

### 5-5: Root formation and soil adaptation

In order to induce roots from the shoots, the shoots were cultured in a MS basal medium containing 20-30 mg/l kanamycin and 200 mg/l cefotaxime. After about 4-5 weeks, roots were formed from the shoots. When the roots grew about 10 cm, the medium was removed and the remaining roots were planted in a zippy pot. They were cultured in an incubator for a few days, and then cultured in a greenhouse. The appearance of transgenic pepper plants obtained one month after the roots were planted in the zippy pot is shown in **FIG. 6****.**

### Example 6: Verification of transgenic plants by PCR assay

Genomic DNAs were extracted from callus expressing GFP and callus expressing no GFP under UV, respectively, among the transgenic plants obtained in Example 5 above. To detect the insertion of a *GFP* gene, each of the extracted DNA templates was subjected to PCR using primers represented by SEQ ID NO: 5 and SEQ ID NO: 6. PCR was performed by repeating 35 cycles, each consisting of 1 minute at 60°C, 1 minute at 94°C and 1 minute at 72°C. As shown in **FIG. 7****,** the PCR assay results showed that a band with a size of about 720bp corresponding to the GFP gene was detected only in the callus expressing GFP.

Furthermore, a genomic DNA was extracted from the leaves of several lines of seedlings regenerated from the callus expressing GFP, and then subjected to PCR in the same manner as described just above. As shown in **FIG. 8****,** the PCR assay results showed that the *GFP* gene was stably introduced into all of P410, P915, P318 and P319 lines. This indicates that the transformation method according to the present invention is not line-specific.

### Example 7: Determination of copy number of GFP gene introduced into transgenic plants

Genomic DNAs were extracted from 14 individuals of the transgenic pepper plants (T0) obtained in Example 5 above, and then subjected to Southern blot analysis, thus determining the copy number of a *GFP* gene inserted into the transgenic plants.

30 µg of each genomic DNA was cut with *Bam*HI and *Xba*I, and then electrophoresed on 0.8% agarose gel at 20 V for 20 hours. Following this, the DNA was transferred to a nitrocellulose membrane, and mixed with a ³²P-dCTP-labeled *GFP* gene and hybridized at 65°C for 16 hours. As shown in **FIG. 9****,** the analysis results showed that two copies of the *GFP* gene existed in the genome of 4 individuals among the 14 individuals, and one copy existed in the remaining individuals. Moreover, the band positions of the transgenic plants were different from each other, thereby suggesting that the callus origins of the transgenic plants are different from each other.

### Example 8: Examination of transformation efficiency

### 8-1: Examination of callus induction rate

The callus induction in 8 lines of pepper plants was attempted in the same manner as in Example 5. The results showed that callus was induced in all the lines at a general rate of about 13%, although there was a difference in callus induction rate between the pepper plant lines (see **Table 6**). Particularly, the case of treatment with 2,4-D or IAA alone showed a higher callus induction rate than that of the case of treatment with a mixture of 2,4-D and zeatin. Furthermore, the callus induction rate of Example 5 was about 10 times higher than that of Example 1 (1.2%; see **Table 2**).

In addition, the ratio of callus expressing GFP under UV was 4.7%, based on total explants used in transformation, and about 36.1% (233/645), based on total callus induced from explants (see **Table 6**). This suggests again that, once callus is induced from explants, transformation rate is greatly increased. The numerical values for each line set forth in **Table 6** represent the total of results for each hormone used in the pre-culture.

**Table 6: Transformation rate according to callus induction rate and GFP expression**

| Pepper lines | Number of explants | Number of callus induced (ratio, %) | Number of callus expressing GFP (ratio, %) |
|---|---|---|---|
| P915 | 1343 | 202(15.0) | 68(5.1) |
| P318 | 853 | 27(3.2) | 9(1.1) |
| P319 | 596 | 186(31.2) | 82(13.8) |
| P409 | 933 | 77(8.3) | 27(2.9) |
| P410 | 402 | 76(18.9) | 19(4.7) |
| P784 | 56 | 5(8.9) | 2(3.6) |
| P2377 | 312 | 43(13.8) | 22(7.1) |
| PMAL | 415 | 29(7.0) | 4(1.0) |
| Total | 4910 | 645(13.1) | 233(4.7) |

### 8-2: Examination of shoot formation rate and transformation rate from callus

The frequency of shoot formation from the callus induced from explants was examined for about 5 months after co-culture with *Agrobacterium,* and the result showed a shoot formation frequency of about 37.7% (see **Table 7**)**.** PCR assay on the shoots formed from callus was performed to examine the introduction rate of a *GFP* gene. The PCR assay results showed that the ratio of shoots having a *GFP* gene insertion among the shoots formed from callus was 45% (**Table 7**). The transformation rate of the shoots with the *GFP* gene was determined to be about 1% (27/2792) based on the total number of explants used in transformation. This value is about 5 times higher than that of Example 1 (0.19%) (see **Table 4**).

**Table 7: Shoot formation rate and transformation rate from callus**

| Pepper lines | Number of shoots formed from the callus | Number of shoots positive in the PCR assay |
|---|---|---|
| P915 | 12/68 | 2 |
| P318 | 8/9 | 4 |
| P319 | 40/82 | 21 |
| Total | 60/159 (37.7%) | 27/60 (45.0%) |

### INDUCTRIAL APPLICABILITY

As described above, in the present invention, the pepper explants were pre-cultured in callus induction medium and then transformed so as to artificially form callus, and the regeneration of plants was induced from the callus. The inventive method shows very high transformation efficiency and can transform pepper plants in a line non-specific manner. Furthermore, the inventive method allows mass-producing various lines of transgenic pepper plants at high efficiency, since the time taken for the preparation of the transgenic pepper plants is shortened as compared to the prior method.

## Claims

1. A method for producing transgenic pepper plants, the method comprising the steps of:
(a) pre-culturing explants of pepper in a callus induction medium;
(b) co-culturing the pre-cultured explants with *Agrobacterium* into which a target gene have been introduced;
(c) culturing the co-cultured explants in a selection medium so as to form callus and selecting the formed callus; and
(d) cutting the callus and culturing the cut callus in a shoot induction medium so as to form shoots.

2. The method according to claim 1, wherein the explants of step (a) are wounded in order to promote callus induction.

3. The method according to claim 1, wherein the explants of step (a) are cotyledons or hypocotyls.

4. The method according to claim 1, wherein the callus induction medium of step (a) comprises one or more selected from the group consisting of zeatin, 2,4-dichlorophenoxy acetic acid (2,4-D), indole-3-acetic acid (IAA), naphthalene acetic acid (NAA) and benzylaminopurine (BA).

5. The method according to claim 4, wherein the callus induction medium of step (a) comprises one or more selected from the group consisting of 0.01-5 mg/l of 2,4-D, 0.01-5 mg/l of IAA, 0.01-5 mg/l of zeatin, 0.01-5 mg/l of NAA and 0.01-5 mg/l BA.

6. The method according to claim 4, wherein the medium comprises 1 mg/l of 2,4-D or 1 mg/l of IAA.

7. The method according to claim 4, wherein the medium comprises a mixture of 0.01-2 mg/l of zeatin with one selected from the group consisting of 0.1-5 mg/l of 2,4-D, 0.1-5 mg/l of IAA and 0.01-2 mg/l of NAA.

8. The method according to claim 1, wherein co-culturing in the step (b) is performed in the same medium as the callus induction medium used in the step (a).

9. The method according to claim 1, wherein the selection medium in the step (c) comprises a mixture of 0.1~5mg/l of zeatin and 0.01∼1mg/l of IAA or a mixture of 0.1~5mg/l of zeatin and 0.01~1mg/l of NAA.

10. The method according to claim 1, wherein the shoot induction medium in the step (d) comprises one selected from the group consisting of:
(i) 0.5∼10mg/l of zeatin;
(ii) a mixture of 0.5∼10mg/l of zeatin and 0.01∼0.2mg/l of IAA; and
(iii) a mixture of 0.5∼10mg/l of zeatin and 0.01~0.2mg/l ofNAA.

11. The method according to claim 1, wherein the following (e) step is additionally comprised after (d) step: (e) transferring the shoots into a root formation medium, and then culturing the transferred shoots to form roots.

12. The method according to claim 1, wherein the target gene is a gene involved in plant defense mechanisms or a gene involved in the biosynthesis of useful metabolites.

13. The method according to claim 12, wherein the gene involved in plant defense mechanisms is a gene encoding a protein selected from the group consisting of ribosome inactivating protein (RIP), jasmonic acid carboxyl methyltransferase, trehalose synthase, plant defensin 1.2, thionine synthase, glucanase, chitinase, phenylalanine ammonia lyase, chalcone synthase, glutathione-S-transferase, anthranilate synthase, storage protein, calmodulin, tryptophan synthase, proteinase inhibitor II, nitric oxide synthase, cystemine, fatty acid peroxidase, allene oxide synthase, pepper-PMMV interaction 1 transcription factor (PPI1), WRKY domain transcription factor, pathogen-responsive protein, and viral coat protein.

14. The method according to claim 13, wherein the gene encoding the viral coat protein is selected from the group consisting of *TMV-CP, CMY-CP* and *PepMoV-CP* gene.

15. The method according to claim 12, wherein the gene involved in the biosynthesis of useful metabolites is selected from the group consisting of genes involved in the biosynthesis of tannin, sinapine, saponin, allicin, spinosin, cinnamic acid, flavonoid, terpenoid, catechin, vitamin, penicilline, indole, insulin, prostaglandin, taxol, alisol, ricin, cartenoid and capsisin.

16. The method according to claim 1, wherein the pepper is genus *Capsicum.*

17. The method according to claim 16, wherein said genus *Capsicum* is *Capsicum annuum* L.

18. The method according to claim 17, wherein said *Capsicum annuum* L is selected from the group consisting of chilli pepper (*Capsicum annuum* L. var. acuminatum), sweet or bell pepper (*Capsicum annuum* L. var. grossum), cone pepper (*Capsicum annuum* L. var. conoides), cherry pepper (*Capsicum annuum* L. var. cerasiforme), red cluster pepper (*Capsicum annuum* L. var. fasciculatum) and long pepper (*Capsicum annuum* L. var. longum).

## Patentansprüche

1. Verfahren zur Herstellung einer transgenen Paprika- oder Chilipflanze, wobei das Verfahren die Schritte von:
(a) Vorkultivieren von Paprika- oder Chili-Explantaten in einem Callus-Induktionsmedium;
(b) Co-Kultivierung der vorkultivierten Explantate mit *Agro-bacterium*-Bakterien, in welche ein Target-Gen eingeführt worden ist;
(c) Kultivieren der co-kultivierten Explantate in einem Selektionsmedium, um einen Callus zu bilden, und Auswählen des gebildeten Callus; und
(d) Abschneiden des Callus und Kultivieren des abgeschnittenen Callus in einem Spross-Induktionsmedium, um Sprosse zu bilden,
umfasst.

2. Verfahren gemäß Anspruch 1, wobei die Explantate von Schritt (a) verletzt werden, um die Callus-Induktion zu fördern.

3. Verfahren gemäß Anspruch 1, wobei die Explantate von Schritt (a) Kotyledonen oder Hypokotyle sind.

4. Verfahren gemäß Anspruch 1, wobei das Callus-Induktionsmedium von Schritt (a) eines oder mehrere, ausgewählt aus der Gruppe, bestehend aus Zeatin, 2,4-Dichlorphenoxyessigsäure (2,4-D), Indol-3-essigsäure (IAA), Naphthalinessigsäure (NAA) und Benzylaminopurin (BA) umfasst.

5. Verfahren gemäß Anspruch 4, wobei das Callus-Induktionsmedium von Schritt (a) eines oder mehrere, ausgewählt aus der Gruppe, bestehend aus 0,01-5 mg/l 2,4-D, 0,01-5 mg/l IAA, 0,01-5 mg/l Zeatin, 0,01-5 mg/l NAA und 0,01-5 mg/l BA umfasst.

6. Verfahren gemäß Anspruch 4, wobei das Medium 1 mg/l 2,4-D oder 1 mg/ml IAA umfasst.

7. Verfahren gemäß Anspruch 4, wobei das Medium eine Mischung von 0,01-2 mg/l Zeatin mit einem, ausgewählt aus der Gruppe, bestehend aus 0,1-5 mg/l 2,4-D, 0,1-5 mg/l IAA und 0,01-2 mg/l NAA umfasst.

8. Verfahren gemäß Anspruch 1, wobei das Co-Kultivieren im Schritt (b) im gleichen Medium durchgeführt wird, welches im Schritt (a) als Callus-Induktionsmedium verwendet wird.

9. Verfahren gemäß Anspruch 1, wobei das Selektionsmedium im Schritt (c) eine Mischung von 0,1∼5 mg/l Zeatin und 0,01-1 mg/l IAA oder eine Mischung von 0,1-5 mg/l Zeatin und 0,01-1 mg/l NAA umfasst.

10. Verfahren gemäß Anspruch 1, wobei das Spross-Induktionsmedium im Schritt (d) eines, ausgewählt aus der Gruppe, bestehend aus:
(i) 0,5-10 mg/l Zeatin;
(ii) eine Mischung von 0,5-10 mg/l Zeatin und 0,01-0,2 mg/l IAA; und
(iii) eine Mischung von 0,5-10 mg/l Zeatin und 0,01-0,2 mg/l NAA
umfasst.

11. Verfahren gemäß Anspruch 1, wobei der folgende (e) Schritt zusätzlich nach Schritt (d) durchgeführt wird: (e) Überführen der Sprosse in ein Wurzelbildungsmedium und danach Kultivieren der überführten Sprosse, um Wurzeln zu bilden.

12. Verfahren gemäß Anspruch 1, wobei das Target-Gen ein Gen ist, welches an Pflanzen-Abwehrmechanismen beteiligt ist oder ein Gen ist, dass an der Biosynthese nützlicher Metabolite beteiligt ist.

13. Verfahren gemäß Anspruch 12, wobei das Gen, welches an Pflanzen-Abwehrmechanismen beteiligt ist, ein Gen ist, welches ein Protein codiert, ausgewählt aus der Gruppe, bestehend aus Ribosom-Inactivating-Protein (RIP), Jasmonsäure-Carboxyl-Methyl-Transferase, Trehalose-Synthase, Pflanzen-Defensin 1.2, Thionin-Synthase, Glucanase, Chitinase, Phenylalanin-Ammonium-Lyase, Chalcon-Synthase, Glutathion-S-Transferase, Anthranilat-Synthase, Speicherprotein, Calmodulin, Tryptophan-Synthase, Proteinaseinhibitor II, Stickoxid-Synthase, Cystemin, Fettsäureperoxidase, Allenoxid-Synthase, Capsicum-PMMV-Interaktion-I-Transkriptionsfaktor (PPI1), WRKY-Domäne-Transkriptionsfaktor, Pathogen-responsives Protein und viralem Hüllprotein.

14. Verfahren gemäß Anspruch 13, wobei das Gen, welches das virale Hüllprotein codiert, ausgewählt ist aus der Gruppe, bestehend aus dem *TMV-CP-,* dem *CMV-CP-* und dem *PepMoV-CP*-Gen.

15. Verfahren gemäß Anspruch 12, wobei das Gen, welches an der Biosynthese nützlicher Metabolite beteiligt ist, ausgewählt wird aus der Gruppe, bestehend aus den Genen, welche an der Biosynthese von Tannin, Sinapin, Saponin, Allicin, Spinosin, Zimtsäure, Flavonoid, Terpenoid, Catechin, Vitamin, Penicillin, Indol, Insulin, Prostaglandin, Taxol, Alisol, Ricin, Carotinoid und Capsaicin beteiligt sind.

16. Verfahren gemäß Anspruch 1, wobei die Paprika- oder Chilipflanze zum Genus *Capsicum* gehört.

17. Verfahren gemäß Anspruch 16, wobei das Genus *Capsicum Capsicum annuum* L. ist.

18. Verfahren gemäß Anspruch 17, wobei das *Capsicum annuum L.* ausgewählt wird aus der Gruppe, bestehend aus Chilipaprika (*Capsicum annuum* L. var. acuminatum), süßem oder Gemüsepaprika (*Capsicum annuum* L. var. grossum), Kegelpaprika (*Capsicum annuum* L. var. conoides), Kirschpaprika (*Capsicum annuum L.* var. cerasiforme), rotem Büschel-Paprika (*Capsicum annuum L.* var. fasciculatum) und Langpaprika (*Capsicum annuum* L. var. longum).

## Revendications

1. Procédé de production de plants de piment transgéniques, le procédé comprenant les étapes suivantes :
(a) pré-culture d'explants de piment dans un milieu d'induction de cals ;
(b) co-culture des explants pré-cultivés avec *Agrobacterium* chez lequel un gène cible a été introduit ;
(c) culture des explants co-cultivés dans un milieu de sélection de façon à former un cal et sélection du cal formé ; et
(d) excision du cal et culture du cal excisé dans un milieu d'induction de pousses de façon à former des pousses.

2. Procédé selon la revendication 1, dans lequel les explants de l'étape (a) sont blessés afin de promouvoir l'induction de cals.

3. Procédé selon la revendication 1, dans lequel les explants de l'étape (a) sont des cotylédons ou des hypocotyles.

4. Procédé selon la revendication 1, dans lequel le milieu d'induction de cals de l'étape (a) comprend un ou plusieurs agents d'induction choisis dans le groupe constitué par la zéatine, l'acide 2,4-dichlorophénoxy-acétique (2,4-D), l'acide indole-3-acétique (IAA), l'acide naphtalène acétique (NAA) et la benzylaminopurine (BA).

5. Procédé selon la revendication 4, dans lequel le milieu d'induction de cals de l'étape (a) comprend un ou plusieurs agents d'induction choisis dans le groupe constitué par 0,01-5 mg/l de 2,4-0, 0,01-5 mg/l d'IAA, 0,01-5 mg/l de zéatine, 0,01-5 mg/l de NAA et 0,01-5 mg/l de BA.

6. Procédé selon la revendication 4, dans lequel le milieu comprend 1 mg/l de 2,4-D ou 1 mg/l d'IAA.

7. Procédé selon la revendication 4, dans lequel le milieu comprend un mélange de 0,01-2 mg/l de zéatine avec un agent d'induction choisi dans le groupe constitué par 0,1-5 mg/l de 2,4-D, 0,1-5 mg/l d'IAA et 0,01-2 mg/l de NAA.

8. Procédé selon la revendication 1, dans lequel la co-culture dans l'étape (b) est réalisée dans le même milieu que le milieu d'induction de cals utilisé dans l'étape (a).

9. Procédé selon la revendication 1, dans lequel le milieu de sélection dans l'étape (c) comprend un mélange de 0,1∼5 mg/l de zéatine et de 0,01∼1 mg/l d'IAA ou un mélange de 0,1~5 mg/l de zéatine et de 0,01~1 mg/l de NAA.

10. Procédé selon la revendication 1, dans lequel le milieu d'induction de pousses dans l'étape (d) comprend un agent d'induction choisi dans le groupe constitué par :
(i) 0,5~10 mg/l de zéatine ;
(ii) un mélange de 0,5~10 mg/l de zéatine et de 0,01~0,2 mg/l d'IAA ; et
(iii) un mélange de 0,5∼10 mg/l de zéatine et de 0,01~0,2 mg/l de NAA.

11. Procédé selon la revendication 1, dans lequel l'étape (e) suivante est comprise en plus après l'étape (d) : (e) transfert des pousses dans un milieu de formation de racines, puis culture des pousses transférées pour former des racines.

12. Procédé selon la revendication 1, dans lequel le gène cible est un gène impliqué dans le mécanisme de défense des plantes ou un gène impliqué dans la biosynthèse de métabolites utiles.

13. Procédé selon la revendication 12, dans lequel le gène impliqué dans le mécanisme de défense des plantes est un gène codant pour une protéine choisie dans le groupe constitué par la protéine d'inactivation des ribosomes (RIP), la carboxyl-méthyltransférase de l'acide jasmonique, la tréhalose synthase, la défensine 1.2 des plantes, la thionine synthase, la glucanase, la chitinase, la phénylalanine ammonia-lyase, la chalcone synthase, la glutathione-S-transférase, l'anthranilate synthase, une protéine de stockage, la calmoduline, la tryptophane synthase, l'inhibiteur II des protéinases, l'acide nitrique synthase, la cystémine, l'acide gras peroxydase, l'oxyde d'allène synthase, le facteur de transcription de l'interaction piment-PMMV 1 (PPI1), le facteur de transcription du domaine WRKY, une protéine sensible aux pathogènes, et une protéine d'enveloppe virale.

14. Procédé selon la revendication 13, dans lequel le gène codant pour la protéine d'enveloppe virale est choisi dans le groupe constitué par le gène *TMV-CP, CMV-CP* et *PepMoV-*CP.

15. Procédé selon la revendication 12, dans lequel le gène impliqué dans la biosynthèse de métabolites utiles est choisi dans le groupe constitué par les gènes impliqués dans la biosynthèse de la tannine, la sinapine, la saponine, l'allicine, la spinosine, l'acide cinnamique, un flavonoïde, un terpénoïde, la catéchine, la vitamine, la pénicilline, l'indole, l'insuline, la prostaglandine, le taxol, l'alisol, le ricin, un caroténoïde et la capsicine.

16. Procédé selon la revendication 1, dans lequel le piment est du genre *Capsicum.*

17. Procédé selon la revendication 16, dans lequel ledit genre *Capsicum* est *Capsicum annuum L.*

18. Procédé selon la revendication 17, dans lequel ledit *Capsicum annuum* L. est choisi dans le groupe constitué par le piment chili *(Capsicum annuum* L. var. acuminatum), le piment doux ou poivron *(Capsicum annuum* L. var. grossum), le piment conique *(Capsicum annuum* L. var. conoides), le piment cerise *(Capsicum annuum* L. var. cerasiforme), le piment à bouquet rouge *(Capsicum annuum* L. var. fasciculatum) et le pili-pili *(Capsicum annuum* L. var. longum).


### Sequence Listing

<110> NONGWOOBIO
<120> METHOD FOR PREPARING TRANSGENIC PEPPER USING CALLUS INDUCTION
<130> PP-B0045 (SEQ)
<150> KR 10-2004-0016722
   <151> 2004-03-12
<160> 6
<170> KopatentIn 1.71
<210> 1
   <211> 20
   <212> DNA
   <213> Artificial Sequence
<220>
   <223> forward primer for PCR
<400> 1
   atgacgcaca atcccactat 20
<210> 2
   <211> 18
   <212> DNA
   <213> Artificial Sequence
<220>
   <223> reverse primer for PCR
<400> 2
   cgaacccctg aaaataat 18
<210> 3
   <211> 20
   <212> DNA
   <213> Artificial Sequence
<220>
   <223> forward primer for PCR
<400> 3
   atgacgcaca atcccactat 20
<210> 4
   <211> 17
   <212> DNA
   <213> Artificial Sequence
<220>
   <223> reverse primer for PCR
<400> 4
   gtaccacttg aagaagc 17
<210> 5
   <211> 20
   <212> DNA
   <213> Artificial Sequence
<220>
   <223> forward primer for PCR
<400> 5
   atgacgcaca atcccactat 20
<210> 6
   <211> 23
   <212> DNA
   <213> Artificial Sequence
<220>
   <223> reverse primer for PCR
<400> 6
   catgtggtct ctcttttcgt tgg 23